(19) Europäisches Patentamt
European Patent Office
Office européen des brevets

(11) Publication number: 0 002 370
B1

# (12) EUROPEAN PATENT SPECIFICATION

(45) Date of publication of patent specification: 11.05.83

(51) Int. Cl.³: C 07 D 498/04 //(C07D498/04, 205/00, 263/00)

(21) Application number: 78300713.1

(22) Date of filing: 01.12.78

## (54) A process for the preparation of ether derivatives of clavulanic acid.

(30) Priority: 02.12.77 GB 5023377

(43) Date of publication of application: 13.06.79 Bulletin 79/12

(45) Publication of the grant of the patent: 11.05.83 Bulletin 83/19

(84) Designated Contracting States: CH DE FR GB IT

(56) References cited:
DE - A - 2 604 697
DE - A - 2 646 004
DE - A - 2 752 701
DE - A - 2 754 763
DE - A - 2 808 116

Survey of Organic Syntheses, John Wiley, Vol 1, pages 305 + 306

HOUBEN-WEYL "Methoden der organischen Chemie" 4. Auflage, Band VI/3, 1965, Thieme Verlag, Stuttgart pages 456 to 457

The file contains technical information submitted after the application was filed and not included in this specification

(73) Proprietor: BEECHAM GROUP PLC
Beecham House Great West Road
Brentford Middlesex (GB)

(72) Inventor: Gasson, Brian Charles
9, Clarence Walk
Redhill Surrey (GB)

(74) Representative: Hesketh, Alan, Dr. et al,
European Patent Attorney Beecham Pharmaceuticals Great Burgh Yew Tree Bottom Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 370 B1

Courier Press, Leamington Spa, England

# A process for the preparation of ether derivatives of clavulanic acid

The present invention relates to a process for the preparation of derivatives of clavulanic acid.

Belgian Patent No: 847045 discloses that esters of ethers of clavulanic acid which are β-lactamase inhibitors and antibacterial agents may be prepared by the etherification of esters of clavulanic acid. We have now found that epoxides may be used in a convenient synthesis to prepare certain such compounds and the corresponding free acids and salts.

Accordingly the present invention provides a process for the preparation of the compounds of the formula (I):

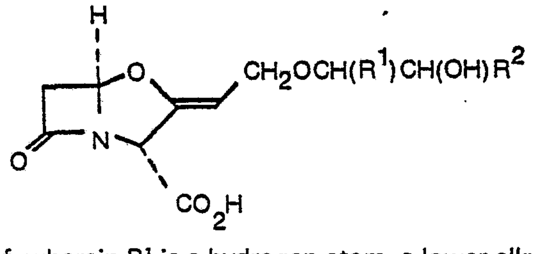

(I)

and salts and esters thereof, wherein $R^1$ is a hydrogen atom, a lower alkyl group, a lower alkenyl group, a phenyl group or a phenyl-(lower alkyl) group; and $R^2$ is a hydrogen atom, a lower alkyl group, or a $CH_3OR^3$ group where $R^3$ is a hydrogen atom, a lower alkyl group, a phenyl group, a phenyl-(lower alkyl) group or a lower acyl group, or $R^1$ is joined to $R^2$ to form the residue of a 5—7 membered saturated carbocyclic ring; which process comprises the reaction in the presence of boron trifluoride etherate of an ester of clavulanic acid with a compound of the formula (II):

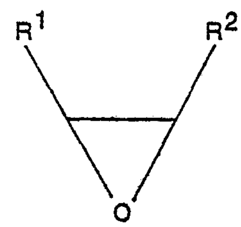

(II)

wherein $R^1$ and $R^2$ are as defined in relation to formula (I), and thereafter, if desired, de-esterifying the thus-produced ester to form the free acid or a salt.

When used herein the term 'lower' means the group contains up to 4 carbon atoms and more suitably up to 3 carbon atoms.

Most suitably $R^1$ is a hydrogen atom.

Most suitably $R^2$ is a hydrogen atom.

Preferably $R^1$ and $R^2$ are both hydrogen atoms so that a particularly favoured process according to this invention comprises the preparation of an ester of 9-O-(2-hydroxyethyl)-clavulanic acid which process comprises the reaction of the corresponding ester of clavulanic acid with ethylene oxide.

When used herein the term ester includes esters notionally derived from an alcohol of the formula ROH where R is an organic residue. Suitable groups R include alkyl, alkenyl, alkynyl, aryl, arylalkyl or other similar groups any of which may be substituted if desired. In order not to increase the molecular weight to an unreasonable extent, group R do not normally include more than 16 carbon atoms, more suitably, not more than 12 carbon atoms and most suitably, not more than 8 carbon atoms.

Preferably, the group R is notionally derived from an alcohol ROH which is pharmaceutically acceptable.

Suitable substituents which may be included in the group R include halogen atoms and lower alkoxyl, hydroxyl, lower acyloxy and like groups. With reference to esterifying groups, the term 'lower' means that the group contains up to 6 carbon atoms, and preferably up to 4 carbon atoms. Thus, for example, R may be a methyl, ethyl, n-propyl, iso-propyl, straight or branched butyl, pentyl, heptyl, octyl, nonyl, decyl, undecyl, dodecyl, vinyl, allyl, butenyl, cyclopropyl, cyclobutyl, cyclopentyl, cyclohexyl, cycloheptyl, cyclohexenyl, cyclohexadienyl, methylcyclopentyl, methylcyclohexyl, benzyl, benzylhydryl, phenylethyl, naphthylmethyl, phenyl, naphthyl, propynyl, tolyl, 2-chloroethyl, 2,2,2-trichloroethyl, 2,2,2-trifluoroethyl, acetylmethyl, benzoylmethyl, 2-methoxyethyl, p-bromobenzyl, m-chlorobenzyl, 6-methoxynaphthyl-2-methyl, p-chlorophenyl, p-methoxyphenyl, p-chlorobenzyl, p-methoxybenzyl or p-nitrobenzyl, as well as those groups which are known from the penicillin or cephalosporin arts to produce esters known to be readily hydrolysed *in vivo* to the parent compound.

Readily hydrolysable esterifying groups include, but are not limited to, those of the sub-formulae (a) and (b):

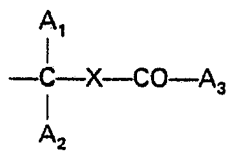 (a) 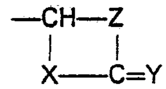 (b)

wherein $A_1$ is a hydrogen atom or an alkyl, aryl or aralkyl group; $A_2$ is a hydrogen atom or methyl group; $A_3$ is an alkyl, aryl or aralkyl group; X is oxygen or sulphur; Y is oxygen or sulphur; and Z is a divalent organic group. Preferred groups of the sub-formulae (a) and (b) include those wherein $A_1$ is a hydrogen atom, $A_2$ is a hydrogen atom or a methyl group and $A_3$ is a methyl, ethyl, propyl, butyl, benzyl, or phenyl group and those wherein X is oxygen, Y is oxygen and Z is $—CH_2CH_2—$, $—CH{:}CH—$,

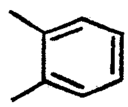 or 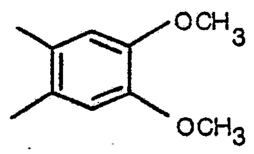 .

When used in conjunction with the preceding formulae, the term 'alkyl' includes alkyl of up to six carbon atoms; the term 'aryl' includes phenyl, naphthyl or phenyl substituted by an inert substituent such as a fluorine or chlorine atom or a methyl or methoxyl group or the like; when used herein the term 'aralkyl' means an alkyl group substituted by an aryl group.

Particularly suitable groups of the sub-formulae (a) and (b) include those of the sub-formulae (c) and (d):

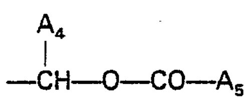 (c) 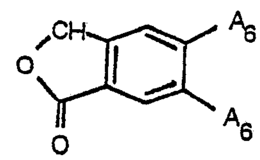 (d)

where $A_4$ is a hydrogen atom or a methyl group, $A_5$ is a methyl, t-butyl or phenyl group and $A_6$ is a hydrogen atom or a methoxyl group.

Esters which undergo some hydrolysis under mild conditions include those where the esterifying group is a group $R^4$ wherein $R^4$ is a hydrocarbon group of 1—9 carbon atoms optionally substituted by halogen, lower alkoxy or hydroxyl.

When used with reference to the group $R^4$ the term 'lower' means that the group contains 1—4 carbon atoms.

Suitable groups $R^4$ include alkyl or aralkyl groups optionally substituted by halogen, methoxyl or hydroxyl.

More suitably alkyl groups $R^4$ are straight-chain groups of up to 6 carbon atoms optionally substituted by one methoxy or hydroxyl group, or one chlorine, bromine or iodine atom or by a $CCl_3$ or $CF_3$ group.

A particularly suitable hydrolysable ester is the methoxymethyl ester.

A further group of particularly suitable ester of this invention are those useful intermediates which are readily converted to the free acid or a salt thereof by chemical or biochemical techniques which are known from the penicillin or cephalosporin arts to be sufficiently mild not to degrade reactive acid-labile $\beta$-lactam rings.

Most suitably, the ester is one removable by hydrogenolysis. Conventional esters for such a process include those wherein the esterifying group is a group $CHR^5R^6$ wherein $R^5$ is a hydrogen atom or an optionally substituted phenyl group and $R^6$ is an optionally substituted phenyl group.

Most suitably $R^5$ is a hydrogen atom or a phenyl, tolyl, chlorophenyl or methoxyphenyl group and $R^6$ is a phenyl, tolyl, chlorophenyl or methoxyphenyl group.

Preferably $R^5$ is a hydrogen atom and $R^6$ is a phenyl group.

By and large, the nature of any substituent in the ester moiety is unimportant as long as it does not interfere with the hydrogenolysis reaction.

The etherification reaction may be carried out at any convenient non-extreme temperature but in general a depressed temperature (for example −10°C to +10°C) is most suitable. The reaction normally employs an organic solvent such as a halohydrocarbon, (for example dichloromethane or chloroform), an ether (for example tetrahydrofuran), or an ester (for example ethyl acetate). The solvent is normally strictly non-hydroxylic.

Suitable methods of de-esterification include those described in British Patent No: 1508977,

DE—A—2 646 004, DE—A—2 754 763 and Belgian Patents Nos: 827926 and 847045. Such methods include mild base hydrolysis (e.g. at pH 8—9.5) of lower alkyl or lower alkoxyalkyl esters and hydrogenolysis of benzyl and substituted benzyl esters.

Hydrogenolysis of benzyl or substituted benzyl esters will normally be effected in the presence of a transition metal catalyst, for example, palladium on charcoal, in an inert solvent, such as tetrahydrofuran, optionally containing water.

When a base is present during the hydrogenolysis a salt of the compound of the formula (I) is produced. Alternatively, the hydrogenolysis may be performed in the absence of a base, and the resultant acid subsequently treated with a base to form a salt.

Suitable bases include sodium and potassium bicarbonate, calcium and magnesium carbonate, and the like.

Most suitably, the de-esterification aspect of the process comprises the preparation of 9-O-(2-hydroxyethyl)-clavulanic acid or a salt thereof by the de-esterification of an ester of 9-O-(2-hydroxyethyl)-clavulanic acid formed by the reaction of an ester of clavulanic acid with ethylene oxide.

The compounds of the formula (I) and their salts and esters are β-lactamase inhibitors, and may be formulated for use in treating bacterial infections as described in Belgian Patent No: 847045.

The following Examples illustrate the invention. The terms Celite and Kieselgel used herein are Registered Trade Marks.

### Example 1
#### Benzyl 9-O-(2-hydroxyethyl)-clavulanate

A solution of benzyl clavulanate (289 mg) and ethylene oxide (100 mg) in methylene chloride (2 ml) was cooled in an ice bath and treated with boron trifluoride etherate (0.02 ml). The mixture was stirred at 0°C for 1½ hours and then allowed to warm to room temperature for ½ hour. The solution was then shaken with excess sodium bicarbonate solution and the organic phase separated. The aqueous phase was extracted twice with chloroform and the combined organic solutions were washed with brine, dried over magnesium sulphate and evaporated. The product was isolated by column chromatography using gradient elution (Kieselgel, ethyl acetate:cyclohexane 1:1 → 2:1) to yield the title compound (68 mg) after evaporation of the solvent.

$\nu_{max}$ (film) 3450, 1805, 1750 and 1695 $cm^{-1}$.

δ ($CDCl_3$) 2.8 (1H, exchangeable), 3.04 (1H, d, J = 17Hz), 3.3—3.8 (5H, m), 4.13 (2H, d, J = 7Hz), 4.87 (1H, broad t, J = 7Hz), 5.13 (1H, broad s), 5.23 (2H, s), 5.72 (1H, d, J = 3Hz), 7.38 (5H, s).

### Example 2
#### Benzyl 9-O-(2-hydroxy-1-phenylethyl)-clavulanate

To a stirred solution of benzyl clavulanate (1 g) and styrene oxide (1 ml) in dry methylene chloride (10 ml) was added boron trifluoride etherate (0.02 ml). The mixture was stirred for 3 hours and then treated with excess sodium bicarbonate solution. The organic phase was separated, washed with brine, dried over magnesium sulphate and evaporated. The product was isolated by column chromatography using gradient elution (Kieselgel, cyclohexane:ethyl acetate 3:1 — 1:1) to yield, after evaporation of the solvent, the title compound (357 mg).

$\nu_{max}$ (film) 3500, 1805, 1750 and 1700 $cm^{-1}$.

δ ($CDCl_3$) 2.28 (1H, exchangeable), 2.94 (1H, d, J = 17Hz), 3.40 (1H, dd, J = 3 and 17Hz), 3.5—3.7 (2H, m), 3.98 (2H, d, J = 7Hz), 4.25—4.5 (1H, m), 4.77 (1H, broad t, J = 7Hz), 4.95 and 5.05 (1H, two singlets due to mixture of diastereoisomers), 5.13 and 5.16 (2H, two singlets), 5.56 (1H, d, J = 3Hz), 7.15—7.35 (10H, m).

### Example 3
#### Benzyl 9-O-(2,3-dihydroxyprop-1-yl)-clavulanate

A solution of benzyl clavulanate (289 mg) and glycidol (0.02 ml) in methylene chloride (2 ml) was cooled in an ice bath and treated with boron trifluoride etherate (0.02 ml). The mixture was stirred at 0°C at 1½ hours and then shaken with excess sodium bicarbonate solution and the organic phase separated. The aqueous phase was extracted with chloroform and the combined organic solutions were washed with brine, dried over magnesium sulphate and evaporated. The product was isolated by column chromatography (Kieselgel, ethyl acetate as eluent) to yield, after evaporation of the solvent, the title compound (27 mg).

$\nu_{max}$ (film) 3400, 1800, 1750 and 1695 $cm^{-1}$.

δ ($CDCl_3$) 2.04 (2H, broad s, exchangeable), 3.02 (1H, d, J = 17Hz), 3.3——3.9 (6H, m), 4.05 (2H, d, J = 7Hz), 4.76 (1H, broad t, J = 7Hz), 5.05 (1H, s), 5.15 (2H, s), 5.64 (1H, d, J = 3Hz), 7.29 (5H, s).

### Example 4
#### Benzyl 9-O-(1-hydroxybut-3-en-2-yl)-clavulanate

A stirred solution of benzyl clavulanate (1 g) and butadiene monoxide (1 ml) in dry methylene chloride (10 ml) was cooled in an ice bath and treated with boron trifluoride etherate (0.02 ml). The

solution was stirred at 0°C for 1 hour and then at room temperature for a further hour. Excess sodium bicarbonate solution was then added and the organic phase separated. The aqueous phase was extracted twice with chloroform and the combined organic solutions were washed with water, dried over magnesium sulphate and evaporated. The product was isolated by column chromatography using gradient elution (Kieselgel, cyclohexane:ethyl acetate 3:1 → 1:1) to yield, after evaporation of the solvent, the title compound (372 mg).

$\nu_{max}$ (film) 3470, 1800, 1750 and 1697 $cm^{-1}$.

$\delta$ ($CDCl_3$) 2.1 (1H, exchangeable), 3.00 (1H, d, J = 17Hz), 3.44 (1H, dd, J = 3 and 17Hz), 3.3—3.6 (2H, m), 3.65—4.2 (3H, m), 4.78 (1H, broad t, J = 7Hz), 5.04 (1H, broad s), 5.14 (2H, s), 5.0—5.4 (2H, m), 5.47—5.84 (1H, m), 5.63 (1H, d, J = 3Hz), 7.29 (5H, s).

## Example 5

### Benzyl 9-O-(2-hydroxycyclohexyl)-clavulanate

A stirred solution of benzyl clavulanate (1.156 g) and cyclohexene oxide (0.8 ml) in methylene chloride (8 ml) was cooled to 0°C and treated with boron trifluoride etherate (0.08 ml). The mixture was stirred at 0°C for 50 minutes and then treated with excess sodium bicarbonate solution. The organic phase was separated and the aqueous phase extracted three times with chloroform. The combined organic phases were washed with brine, dried over magnesium sulphate and evaporated. The product was isolated by column chromatography using gradient elution (Kieselgel, ethyl acetate:cyclohexane 1:3 → 1:1) to yield, after evaporation of the solvent, benzyl 9-O-(2-hydroxycyclohexyl)-clavulanate (308 mg):

$\nu_{max}$ (film) 3450, 1800, 1747 and 1695 $cm^{-1}$.

$\delta$ ($CDCl_3$) 0.9—2.2 (8H, m), 2.63 (1H, broad s, exchangeable), 2.8—3.6 (2H, m), 2.99 (1H, d, J = 17Hz), 3.43 (1H, dd, J = 3 and 17Hz), 3.85—4.35 (2H, m), 4.79 (1H, broad t, J = 7Hz), 5.04 (1H, s), 5.14 (2H, s), 5.63 (1H, d, J = 3Hz), 7.28 (5H, s).

## Example 6

### Methoxymethyl 9-O-(2-hydroxyethyl)-clavulanate

A stirred solution of methoxymethyl clavulanate (486 mg) and ethylene oxide (200 mg) in dry methylene chloride (2 ml) was cooled in an ice bath and treated with boron trifluoride etherate (0.04 ml). After 1½ hours the solution was shaken with excess sodium bicarbonate solution. The organic phase was separated and the aqueous phase extracted with two portions of chloroform. The combined extracts were washed with brine, dried over magnesium sulphate and evaporated. The product was isolated by column chromatography (Kieselgel, gradient elution, ethyl acetate:cyclohexane 1:1 → pure ethyl acetate) to yield, after evaporation of the solvent, the title compound (69 mg).

$\nu_{max}$ (film) 3460, 1805, 1755 and 1695 $cm^{-1}$.

$\delta$ ($CDCl_3$) 2.45 (1H, broad s, exchangeable), 3.03 (1H, d, J = 17Hz), 3.3—3.8 (8H, m), 4.09 (2H, s, J = 7Hz), 4.86 (1H, broad t, J = 7Hz), 5.05 (1H, s), 5.21 (1H, d, J = 6Hz), 5.31 (1H, d, J = 6Hz), 5.66 (1H, d, J = 3Hz).

## Example 7

### Sodium 9-O-(2-hydroxyethyl)-clavulanate

A solution of benzyl 9-O-(2-hydroxyethyl)-clavulanate (200 mg) and sodium bicarbonate (50 mg) in tetrahydrofuran (7.5 ml) and water (1.5 ml) was hydrogenated over 10% palladium/charcoal (80 mg) for 45 minutes. The solution was filtered through Celite and the filter cake washed with water. The combined filtrates were evaporated on a rotary evaporator to remove most of the tetrahydrofuran. The aqueous solution was extracted twice with ethyl acetate, filtered through celite and freeze-dried to yield the title compound (94 mg).

$\nu_{max}$ (KBr) 1790, 1695 and 1615 $cm^{-1}$.

$\delta$ ($D_2O$) 3.05 (1H, d, J = 17Hz), 3.4—3.75 (5H, m), 4.11 (2H, d, J = 8Hz), 4.87 (1H, broad t, J = 8Hz), 4.93 (1H, s), 5.70 (1H, d, J = 3Hz).

## Example 8

### Sodium 9-O-(2-hydroxycyclohexyl)-clavulanate

A solution of benzyl 9-O-(2-hydroxycyclohexyl)-clavulanate (297 mg) in tetrahydrofuran (8 ml) was hydrogenated over 10% palladium/charcoal (100 mg) for 45 minutes. The solution was filtered through Celite and the filter cake washed with tetrahydrofuran. The combined filtrates were diluted with water and treated with a solution of sodium bicarbonate (64.4 mg) in water. Most of the tetrahydrofuran was then removed under vacuum. The aqueous solution was washed three times with ethyl acetate, filtered through Celite and freeze-dried. The product was purified by column chromatography (Kieselgel, 4:1:1 n-butanol:ethanol:water as eluent) to yield, after evaporation of the solvent, the title compound (150 mg).

$\nu_{max}$ (KBr) 1785, 1693, 1613 $cm^{-1}$.

$\delta$ ($D_2O$) 0.70—2.20 (8H, m), 3.02 (1H, d, J = 17Hz), 3.51 (1H, dd, J = 3 and 17Hz), 2.9—2.7 (2H, m), 4.16 (2H, d, J = 8Hz), 4.87 (1H, broad t, J = 8Hz), 4.90 (1H, s), 5.69 (1H, d, J = 3Hz).

Example 9

Sodium 9-O-(2-hydroxy-1-phenylethyl)-clavulanate

A solution of benzyl 9-O-(2-hydroxy-1-phenylethyl)-clavulanate (321 mg) and sodium bicarbonate (65 mg) in a mixture of tetrahydrofuran (12.5 ml) and water (1.5 ml) was hydrogenated over 10% palladium/charcoal (150 mg) for 1 hour. The solution was filtered through Celite and the filter cake washed with water. The combined filtrates were evaporated to remove most of the tetrahydrofuran and the aqueous solution was washed three times with ethyl acetate and freeze-dried to yield the title compound (171 mg).

$\nu_{max}$ (KBr) 1787, 1695 and 1620 $cm^{-1}$.

$\delta$ ($D_2O$) 2.91 (1H, d, J = 17Hz), 3.47 (1H, dd, J = 3 and 17Hz), 3.64 (2H, d, J = 6Hz), 4.02 (2H, d, J = 7Hz), 4.4—5.0 (3H, m), 5.57 (1H, d, J = 3Hz), 7.34 (5H, s).

EXAMPLE 10

Sodium 9-O-(2,3-dihydroxyprop-1-yl)-clavulanate

A solution of benzyl 9-O-(2,3, dihydroxyprop-1-yl)-clavulanate (299 mg) in tetrahydrofuran (10 ml) was hydrogenated over 10% palladium/charcoal (100 mg) for 25 mins. The solution was filtered through Celite and the filter cake washed with tetrahydrofuran. The filtrate was diluted with water and treated with a solution of sodium bicarbonate (69.1 mg) in water. Most of the tetrahydrofuran was then evaporated in a rotary evaporator and the aqueous solution was extracted three times with ethyl acetate. The aqueous solution was then filtered through Celite, evaporated, and the residue dried over phosphorus pentoxide to yield the title compound (169 mg).

$\nu_{max}$ (KBr) 1778, 1690 and 1610 $cm^{-1}$.

$\delta$ ($D_2O$) 3.08 (1H, d, J = 17Hz), 3.3—4.0 (6H, m), 4.19 (2H, d, J = 7Hz), 4.88 (1H, broad t, J = 7Hz), 4.93 (1H, s), 5.70 (1H, d, J = 3Hz).

Example 11

Benzyl 9-O-(3-benzyloxy-2-hydroxyprop-1-yl)-clavulanate

A solution of benzyl clavulanate (2.5 gm) and 3-benzyloxyepoxypropane (1.25 gm) in methylene chloride (20 ml) was cooled to −30° and treated with boron trifluoride etherate (0.2 ml). The mixture was stirred at −30° to −10° for 6 hrs and then treated with excess sodium bicarbonate solution. The organic phase was separated, washed with sodium chloride solution, dried over sodium sulphate and evaporated. Chromatography of the residue (Kieselgel, ethyl acetate-petroleum ether as eluent) gave the desired product (200 mg).

$\nu_{max}$ (film) 3430, 1805, 1750 and 1698 $cm^{-1}$.

$\delta$ ($CDCl_3$) 2.42 (1H, broad s), 2.97 (1H, d, J = 17Hz), 3.40—3.60 (5H, m), 3.92 (1H, m), 4.05 (2H, d, J = 7Hz), 4.52 (2H, s), 4.78 (1H, t, J = 7Hz), 5.07 (1H, s), 5.15 (2H, s), 5.65 (1H, d, J = 3Hz), 7.30 (10H, s).

Example 12

Methyl 9-O-(2-hydroxyethyl)-clavulanate

A stirred solution of methyl clavulanate (426 mg), and ethylene oxide (200 mg) in methylene chloride (4 ml) was cooled in an ice bath and treated with boron trifluoride etherate (0.04 ml). The mixture was stirred at 0° for 2 hrs and then allowed to warm to room temperature for 1 hr. Excess sodium bicarbonate solution was then added and the organic phase separated. The aqueous phase was extracted twice with chloroform and the combined organic phases were washed with brine, dried over magnesium sulphate and evaporated. The product was isolated by column chromatography of the residue (Kieselgel, 1:1 ethyl acetate:cyclohexane going to neat ethyl acetate as eluent). Yield: 77 mg.

$\nu_{max}$ (film) 3480, 1805, 1750, 1700 $cm^{-1}$.

$\delta$ ($CDCl_3$) 2.50 (1H, broad), 2.97 (1H, d, J = 17Hz), 3.3—3.85 (5H, m), 3.75 (3H, s), 4.08 (2H, d, J = 7Hz), 4.81 (1H, broad t, J = 7Hz), 5.03 (1H, broad s), 5.65 (1H, d, J = 3Hz).

**Claims**

1. A process of the preparation of a compound of the formula (I):

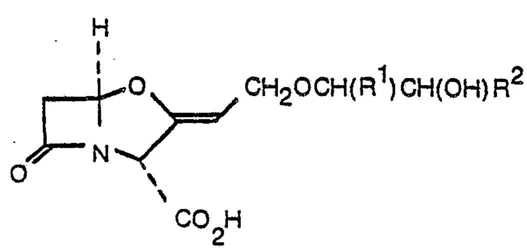

(I)

or a salt or ester thereof wherein R¹ is a hydrogen atom, a lower alkyl group, a lower alkenyl group, a

phenyl group or a phenyl-(lower alkyl) group; and $R^2$ is a hydrogen atom, a lower alkyl group, or a $CH_2OR^3$ group where $R^3$ is a hydrogen atom, a lower alkyl group, a phenyl group, a phenyl-(lower alkyl) group or a lower acyl group, or $R^1$ is joined to $R^2$ to form the residue of a 5—7 membered saturated carbocyclic ring; which process comprises the reaction in the presence of boron trifluoride etherate of an ester of clavulanic acid with a compound of the formula (II):

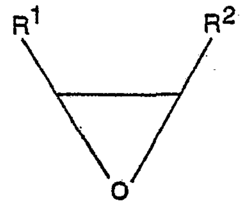

(II)

wherein $R^1$ and $R^2$ are as defined in relation to formula (I), and thereafter, if desired, de-esterifying the thus-produced ester to form the free acid or a salt.

2. A process as claimed in claim 1 wherein $R^1$ is a hydrogen atom.

3. A process as claimed in claim 1 or claim 2 wherein $R^2$ is a hydrogen atom.

4. A process for the preparation of an ester of 9-O-(2-hydroxyethyl)-clavulanic acid, which process comprises the reaction in the presence of boron trifluoride etherate of the corresponding ester of clavulanic acid with ethylene oxide.

5. A process as claimed in any of claims 1—4 wherein the ester of clavulanic acid is notionally derived from an alcohol of the formula ROH where R is an optionally substituted alkyl, alkenyl, aryl or arylalkyl group.

6. A process as claimed in claim 5 wherein the alcohol ROH is pharmaceutically acceptable.

7. A process as claimed in claim 5 or claim 6 wherein the group R is substituted by halogen atoms, lower alkoxyl, hydroxyl or lower acyloxy groups.

8. A process as claimed in any of claims 5—7 wherein R is methyl, ethyl, n- or iso-propyl, straight or branched butyl, pentyl, heptyl, octyl, nonyl, decyl, undecyl, dodecyl, vinyl, allyl, butenyl, cyclopropyl, cyclobutyl, cyclopentyl, cyclohexyl, cycloheptyl, cyclohexenyl, cyclohexadienyl, methylcyclopentyl, methylcyclohexyl, benzyl, benzhydryl, phenylethyl, naphthylmethyl, phenyl, naphthyl, propynyl, tolyl, 2-chloroethyl, 2,2,2-trichloroethyl, 2,2,2-trifluoroethyl, acetylmethyl, benzoylmethyl, 2-methoxyethyl, p-chlorobenzyl, p-methoxybenzyl, p-nitrobenzyl, p-bromobenzyl, p-chlorophenyl, m-chlorobenzyl, 6-methoxynaphthyl-2-methyl or p-methoxyphenyl group.

9. A process as claimed in any of claims 1—4 wherein the esterifying group is a group of the sub-formula (a) or (b):

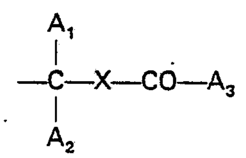

(a)

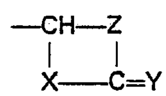

(b)

wherein $A_1$ is a hydrogen atom or an alkyl, aryl or aralkyl group; $A_2$ is a hydrogen atom or methyl group; $A_3$ is an alkyl, aryl or aralkyl group; X is oxygen or sulphur; Y is oxygen or sulphur and Z is a divalent organic group.

10. A process as claimed in claim 9 wherein $A_1$ is a hydrogen atom; $A_2$ is a hydrogen atom or a methyl group; $A_3$ is a methyl, ethyl, propyl, butyl, benzyl, or phenyl group; X is oxygen; Y is oxygen; and Z is $—CH_2CH_2—$, —CH:CH—,

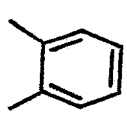

or

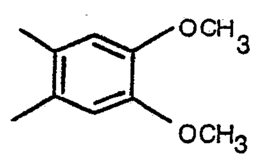

.

11. A process as claimed in claim 9 wherein the group of the sub-formula (a) or (b) is a group of the sub-formula (c) or (d):

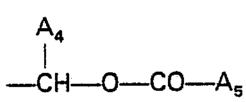

(c)

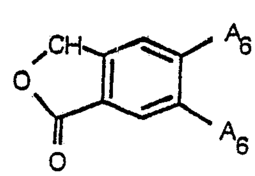

(d)

wherein $A_4$ is a hydrogen atom or methyl group, $A_5$ is a methyl, t-butyl or phenyl group and $A_6$ is a hydrogen atom or a methoxy group.

12. A process as claimed in any of claims 1—4 wherein the esterifying group is a group $R^4$ wherein $R^4$ is a hydrocarbon group of 1—9 carbon atoms optionally substituted by halogen, lower alkoxy or hydroxyl.

13. A process as claimed in claim 12 wherein $R^4$ is an alkyl or aralkyl group optionally substituted by halogen, methoxyl or hydroxyl.

14. A process as claimed in claim 13 wherein $R^4$ is a straight-chain group of up to 6 carbon atoms optionally substituted by one methoxy or hydroxyl group or by one chlorine, bromine or iodine atom or by a $CCl_3$ or $CF_3$ group.

15. A process as claimed in any of claims 1—4 wherein the esterifying group is a group $CHR^5R^6$ wherein $R^5$ is a hydrogen atom or an optionally substituted phenyl group and $R^6$ is an optionally substituted phenyl group.

16. A process as claimed in claim 15 wherein $R^5$ is a hydrogen atom or a phenyl, tolyl, chlorophenyl or methoxyphenyl group and $R^6$ is a phenyl, tolyl, chlorophenyl or methoxyphenyl group.

17. A process as claimed in claim 15 wherein $R^5$ is a hydrogen atom and $R^6$ is a phenyl group.

18. A process for the preparation of 9-O-(2-hydroxyethyl)-clavulanic acid or a salt thereof which process comprises the de-esterification of an ester of 9-O-(2-hydroxyethyl)-clavulanic acid formed by the reaction in the presence of boron trifluoride etherate of an ester of clavulanic acid with ethylene oxide.

**Patentansprüche**

1. Ein Verfahren zur Herstellung einer Verbindung der Formel (I):

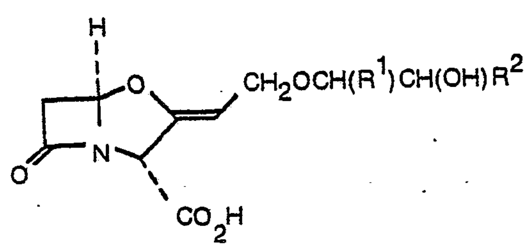

(I)

oder eines Salzes oder Esters derselben, wobei $R^1$ ein Wasserstoffatom, eine niedere Alkylgruppe, eine niedere Alkenylgruppe, eine Phenylgruppe oder eine Phenyl-(niedere Alkyl)-Gruppe ist; und $R^2$ ein Wasserstoffatom, eine niedere Alkylgruppe oder eine $CH_2OR^3$-Gruppe ist, in der $R^3$ ein Wasserstoffatom, eine niedere Alkylgruppe, eine Phenylgruppe, eine Phenyl-(niedere Alkyl)-Gruppe oder eine niedere Acylgruppe ist, oder $R^1$ und $R^2$ zusammen den Rest eines 5- bis 7-gliedrigen gesättigten carbocyclischen Rings bilden, umfassend die Maßnahmen der Reaktion in Gegenwart von Bor-trifluorid-ätherat eines Clavulansäure-esters mit einer Verbindung der Formel (II):

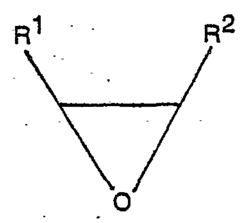

(II)

in der $R^1$ und $R^2$ wie in bezug auf Formel (I) definiert sind, und anschließend, wenn erwünscht, Verseifen des so gebildeten Esters zu der freien Säure oder einem Salz.

2. Ein Verfahren wie in Anspruch 1 beansprucht, wobei $R^1$ ein Wasserstoffatom ist.

3. Ein Verfahren wie in Anspruch 1 oder Anspruch 2 beansprucht, wobei $R^2$ ein Wasserstoffatom ist.

4. Ein Verfahren zur Herstellung eines Esters der 9-O-(2-Hydroxyäthyl)-clavulansäure, umfassend die Maßnahme der Reaktion in Gegenwart von Bor-trifluorid-ätherat des entsprechenden Clavulansäure-esters mit Äthylenoxid.

5. Ein Verfahren wie in einem der Ansprüche 1 bis 4 beansprucht, wobei der Clavulansäure-ester sich begrifflich von einem Alkohol der Formel ROH ableitet, in der R eine gleichfalls substituierte Alkyl-, Alkenyl, Aryl- oder Arylalkylgruppe ist.

6. Ein Verfahren wie in Anspruch 5 beansprucht, wobei der Alkohol ROH pharmakologisch verträglich ist.

7. Ein Verfahren wie in Anspruch 5 oder 6 beansprucht, wobei die Gruppe R mit Halogenatomen, niederen Alkoxy-, Hydroxyl- oder niederen Acyloxygruppen substituiert ist.

8. Ein Verfahren wie in einem der Ansprüche 5 bis 7 beansprucht, wobei R die Methyl-, Äthyl-, n- oder iso-Propyl-, geradkettige oder verzweigte Butyl-, Pentyl-, Heptyl-, Octyl-, Nonyl-, Decyl-, Undecyl-, Dodecyl-, Vinyl-, Allyl-, Butenyl-, Cyclopropyl-, Cyclobutyl-, Cyclopentyl-, cyclohexyl-, Cycloheptyl-, Cyclohexenyl-, Cyclohexadienyl-, Methylcyclopentyl-, Methylcyclohexyl-, Benzyl-, Benzhydryl-, Phenyläthyl-, Naphthylmethyl-, Phenyl-, Naphthyl-, Propinyl-, Tolyl-, 2-Chloräthyl-, 2,2,2-Trichloräthyl-, 2,2,2-Trifluoräthyl-, Acetylmethyl-, Benzoylmethyl-, 2-Methoxyäthyl-, p-Chlorbenzyl-, p-Methoxybenzyl-, p-Nitrobenzyl-, p-Brombenzyl-, p-Chlorbenzyl-, m-Chlorbenzyl-, 6-Methoxynaphthyl-2-methyl- oder p-Methoxyphenyl-Gruppe ist.

9. Ein Verfahren wie in einem der Ansprüche 1 bis 4 beansprucht, wobei die veresternde Gruppe eine Gruppe der Unterformeln (a) oder (b) ist:

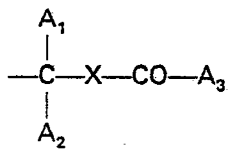 (a) 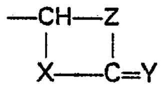 (b)

in denen $A_1$ ein Wasserstoffatom oder eine Alkyl-, Aryl- oder Arylalkylgruppe ist; $A_2$ ein Wasserstoffatom oder eine Methylgruppe ist; $A_3$ eine Alkyl-, Aryl- oder Arylalkylgruppe ist; X Sauerstoff oder Schwefel ist; Y Sauerstoff oder Schwefel ist und Z eine divalente organische Gruppe ist.

10. Ein Verfahren wie in Anspruch 9 beansprucht, wobei $A_1$ ein Wasserstoffatom ist; $A_2$ ein Wasserstoffatom oder eine Methylgruppe ist; $A_3$ eine Methyl-, Äthyl-, Propyl-, Butyl-, Benzyl- oder Phenylgruppe ist; X Sauerstoff ist; Y Sauerstoff ist; und Z $-CH_2CH_2-$, $-CH{:}CH-$,

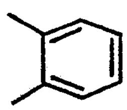 oder 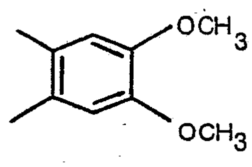

ist.

11. Ein Verfahren wie in Anspruch 9 beansprucht, wobei die Gruppe der Unterformeln (a) oder (b) eine Gruppe der Unterformeln (c) oder (d) ist:

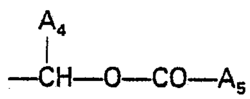 (c) 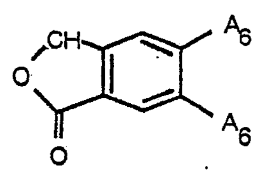 (d)

in denen $A_4$ ein Wasserstoffatom oder eine Methylgruppe ist, $A_5$ eine Methyl-, tert.-Butyl- oder Phenylgruppe ist, und $A_6$ ein Wasserstoffatom oder eine Methoxygruppe ist.

12. Ein Verfahren wie in einem der Ansprüche 1 bis 4 beansprucht, wobei die veresternde Gruppe eine Gruppe $R^4$ ist, in der $R^4$ eine Kohlenwasserstoffgruppe mit 1 bis 9 Kohlenstoffatomen ist, die gegebenenfalls mit Halogenatomen, niederen Alkoxy- oder Hydroxylgruppen substituiert ist.

13. Ein Verfahren wie in Anspruch 12 beansprucht, wobei $R^4$ eine Alkyl- oder Arylalkylgruppe ist, die gegebenenfalls mit Halogenatomen, Methoxy- oder Hydroxylgruppen substituiert ist.

14. Ein Verfahren wie in Anspruch 13 beansprucht, wobei $R^4$ eine geradkettige Gruppe mit bis zu 6 Kohlenstoffatomen ist, die gegebenenfalls mit einer Methoxy- oder Hydroxylgruppe, oder mit einem Chlor-, Brom- oder Jodatom, oder mit einer $CCl_3$- oder $CF_3$- Gruppe substituiert ist.

15. Ein Verfahren wie in einem der Ansprüche 1 bis 4 beansprucht, wobei die veresternde Gruppe eine Gruppe $CHR^5R^6$ ist, in der $R^5$ ein Wasserstoffatom oder eine gegebenenfalls substituierte Phenylgruppe ist und $R^6$ eine gegebenenfalls substituierte Phenylgruppe ist.

16. Ein Verfahren wie in Anspruch 15 beansprucht, wobei $R^5$ ein Wasserstoffatom oder eine Phenyl-, Tolyl-, Chlorphenyl- oder Methoxyphenylgruppe ist, und $R^6$ eine Phenyl-, Tolyl-, Chlorphenyl- oder Methoxyphenylgruppe ist.

17. Ein Verfahren wie in Anspruch 15 beansprucht, wobei $R^5$ ein Wasserstoffatom und $R^6$ eine Phenylgruppe ist.

18. Ein Verfahren zur Herstellung von 9-O-(2-Hydroxyäthyl)-clavulansäure oder eines Salzes derselben, umfassend die Maßnahmen der Verseifung eines Esters der 9-O-(2-Hydroxyäthyl)clavulansäure, der in Gegenwart von Bortrifluorid-ätherat durch Umsetzen eines Clavulansäure-esters mit Äthylenoxid hergestellt worden ist.

**Revendications**

1. Procédé pour la préparation d'un composé de formule (I):

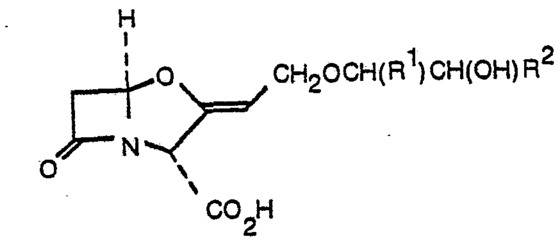

(I)

ou d'un sel ou ester de celui-ci, formule dans laquelle $R^1$ est un atome d'hydrogène, un groupe alcoyle inférieur, un groupe alcényle inférieur, un groupe phényle ou un groupe phényl-alcoyle inférieur; et $R^2$ est un atome d'hydrogène, un groupe alcoyle inférieur ou un groupe $CH_2OR^3$, où $R^3$ est un atome d'hydrogène, un groupe alcoyle inférieur, un groupe phényle, un phényl-alcoyle inférieur ou un groupe acyle inférieur, ou bien $R^1$ est réuni à $R^2$ pour former le reste d'un carbocycle saturé à 5—7 chaînons; ce procédé consistant à effectuer la réaction, en présence d'éthérate de trifluorure de bore, d'un ester d'acide clavulanique avec un composé de formule (II):

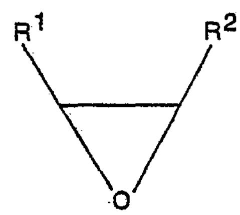

(II)

dans laquelle $R^1$ et $R^2$ ont la même signification que dans le cas de la formule (I), puis si désiré à désestérifier l'ester ainsi produit pour former l'acide libre ou un sel.

2. Procédé suivant la revendication 1, caractérisé en ce que $R^1$ est un atome d'hydrogène.

3. Procédé suivant la revendication 1 ou 2, caractérisé en ce que $R^2$ est un atome d'hydrogène.

4. Procédé pour la préparation d'un ester d'acide 9-O-(2-hydroxyéthyl)-clavulanique, ce procédé consistant à efectuer la réaction, en présence d'éthérate de trifluorure de bore, de l'ester correspondant d'acide clavulanique avec de l'oxyde d'éthylène.

5. Procédé suivant l'une quelconque des revendications 1 à 4, caractérisé en ce que l'ester d'acide clavulanique est en principe dérivé d'un alcool de formule ROH, où R est un groupe alcoyle, alcényle, aryle ou aralcoyle facultativement substitué.

6. Procédé suivant la revendication 5, caractérisé en ce que l'alcool ROH est pharmaceutiquement acceptable.

7. Procédé suivant la revendication 5 ou 6, caractérisé en ce que le groupe R est substitué par des atomes d'halogène ou des groupes alcoxy inférieur, hydroxy ou acyloxy inférieur.

8. Procédé suivant l'une quelconque des revendications 5 à 7, caractérisé en ce que R est un groupe méthyle, éthyle, n- ou iso-propyle, ou sous forme linéaire ou ramifiée, butyle, pentyle, heptyle, octyle, nonyle, décyle, undécyle, dodécyle, vinyle, allyle, buténylе, cyclopropyle, cyclobutyle, cyclopentyle, cyclohexyle, cycloheptyle, cyclohexényle, cyclohexadiényle, méthylcyclopentyle, méthylcyclohexyle, benzyle, benzhydryle, phényléthyle, naphtylméthyle, phényle, naphtyle, propynyle, tolyle, 2-chloréthyle, 2,2,2-trichloréthyle, 2,2,2-trifluoréthyle, acétylméthyle, benzoylméthyle, 2-méthoxyéthyle, p-chlorobenzyle, p-méthoxybenzyle, p-nitrobenzyle, p-bromobenzyle, p-chlorophényle, m-chlorobenzyle, 6-méthoxynaphtyl-2-méthyle ou p-méthoxyphényle.

9. Procédé suivant l'une quelconque des revendications 1 à 4, caractérisé en ce que le groupe d'estérification est un groupe de sous-formules (a) ou (b):

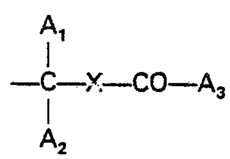 (a)

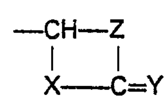 (b)

dans lesquelles $A_1$ est un atome d'hydrogène ou un groupe alcoyle, aryle ou aralcoyle; $A_2$ est un atome d'hydrogène ou un groupe méthyle; $A_3$ est un groupe alcoyle, aryle ou aralcoyle; X est de l'oxygène ou du soufre; Y est de l'oxygène ou du soufre; et Z est un groupe organique bivalent.

10. Procédé suivant la revendication 9, caractérisé en ce que $A_1$ est un atome d'hydrogène; $A_2$ est

un atome d'hydrogène ou un groupe méthyle; $A_3$ est un groupe méthyle, éthyle, propyle, butyle, benzyle ou phényle; X est de l'oxygène; Y est de l'oxygène; et Z est $-CH_2CH_2-$ $-CH:CH-$,

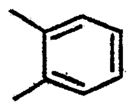

ou

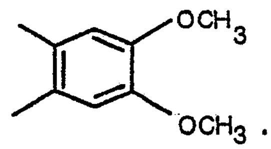

11. Procédé suivant la revendication 9, caractérisé en ce que le groupe de sous-formules (a) ou (b) est un groupe de sous-formule (c) ou (d):

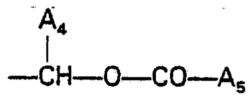

(c)

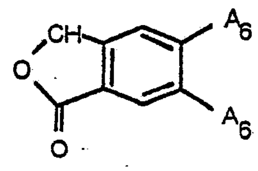

(d)

dans lesquelles $A_4$ est un atome d'hydrogène ou un groupe méthyle, $A_5$ est un groupe méthyle, t-butyle ou phényle, et $A_6$ est un atome d'hydrogène ou un groupe méthoxy.

12. Procédé suivant l'une quelconque des revendications 1 à 4, caractérisé en ce que le groupe d'estérification est un groupe $R^4$ dans lequel $R^4$ est un groupe hydrocarboné ayant de 1 à 9 atomes de carbone, facultativement substitué par un halogène, un alcoxy inférieur ou un hydroxy.

13. Procédé suivant la revendication 12, caractérisé en ce que $R^4$ est un groupe alcoyle ou aralcoyle facultativement substitué par un halogène, un méthoxy ou un hydroxy.

14. Procédé suivant la revendication 13, caractérisé en ce que $R^4$ est un groupe à chaîne linéaire ayant jusqu'à 6 atomes de carbone, facultativement substitué par un groupe méthoxy ou hydroxy ou par un atome de chlore, de brome ou d'iode, ou encore par un groupe $CCl_3$ ou $CF_3$.

15. Procédé suivant l'une quelconque des revendications 1 à 4, caractérisé en ce que le groupe d'estérification est un groupe $CHR^5R^6$ dans lequel $R^5$ est un atome d'hydrogène ou un groupe phényle facultativement substitué et $R^6$ est un groupe phényle facultativement substitué.

16. Procédé suivant la revendication 15, caractérisé en ce que $R^5$ est un atome d'hydrogène ou un groupe phényle, tolyle, chlorophényle ou méthoxyphényle, et $R^6$ est un groupe phényle, tolyle, chlorophényle ou méthoxyphényle.

17. Procédé suivant la revendication 15, caractérisé en ce que $R^5$ est un atome d'hydrogène et $R^6$ est un groupe phényle.

18. Procédé pour la préparation d'acide 9-O-(2-hydroxyéthyl)-clavulanique ou d'un sel de celui-ci, ce procédé consistant à effectuer la désestérification d'un ester d'acide 9-O-(2-hydroxyéthyl)-clavulanique formé par réaction, en présence d'éthérate de trifluorure de bore, d'un ester d'acide clavulanique avec de l'oxyde d'éthylène.